# EUROPEAN PATENT SPECIFICATION

(11) **EP 0 970 360 B1**
(45) Date of publication and mention of the grant of the patent: **24.07.2002**
(21) Application number: 98909877.7
(22) Date of filing: 18.03.1998
(51) Int. Cl.: G01M 15/00, G01N 1/24

(54) **METHOD AND APPARATUS FOR SEPARATING A PARTIAL FLOW FROM A FLOW OF GASES**
VERFAHREN UND VORRICHTUNG ZUR ABTRENNUNG EINES TEILSTROMS AUS EINEM GASFLUSS
PROCEDE ET DISPOSITIF DE SEPARATION D'UN FLUX PARTIEL A PARTIR D'UN ECOULEMENT DE GAZ

(30) Priority: 21.03.1997 NL 1005605; 23.12.1997 NL 1007885
(43) Date of publication of application: 12.01.2000
(73) Proprietor: Van Dijk, Sybren, 8831 XT Winsum (NL); Van Dijk Technics Worldwide N.V., Curaçao (AN)
(72) Inventor: Van Dijk, Sybren, 8831 XT Winsum (NL); Van Dijk Technics Worldwide N.V., Curaçao (AN)
(74) Representative: Ottevangers, Sietse Ulbe
(86) International application number: NL9800155
(87) International publication number: WO9843061

(56) References cited:
- EP-A- 0 155 793
- DE-U- 9 313 492

## Description

The invention relates to a method for separating, by means of a probe, a partial flow from a flow of gases coming from an exhaust pipe of an engine such as a diesel engine for a vehicle, wherein extraction means are present for receiving the flow of gases and discharging it to a location remote from the exhaust pipe, and to an apparatus for carrying out such method.

Such method and apparatus are used inter alia for measuring the CO-content and the soot content of the exhaust gases of a motor vehicle. Such measuring is usually performed in a workshop, so that it is important to collect the exhaust gases and discharge them into the open air. For measuring the CO-content, it is conventional to sealingly couple a collecting hose to the exhaust pipe of the vehicle, which hose is connected to measuring equipment and to an extraction apparatus, so that the exhaust gases cannot end up in the working environment. Such method of operation is not possible for measuring the soot content, because this should take place under "free" pressure conditions, i.e. without the extraction apparatus affecting the pressure of the exhaust gases. For this reason, the soot measuring is effected by. inserting a probe into the exhaust pipe and arranging an extraction hood at some distance behind the exhaust pipe, as is known from DE-U-9313492. However, it turned out that in this manner of operating, a part of the exhaust gases is not discharged via the extraction hood and ends up in the working environment. Moreover, the installation of a loose extraction hood is quite often forgotten, consciously or unconsciously. This may easily involve unpleasant and, most certainly, unhealthy working conditions. Further, the probe, inserted loosely into the exhaust pipe, is in an unstable position. Vibrations, in combination with exhaust gas flowing past, may cause ejection of the probe and may have an adverse effect on the measuring.

EP-A-0 155 793 discloses an exhaust sampler system sealingly connectable to an exhaust pipe, but the exhaust gases are diluted with fresh air, i.e."free" pressure conditions are not present.

The object of the invention is to improve the method described in the opening paragraph in such a manner that the above-described drawbacks no longer occur.

This object is realized by the invention in that the exhaust pipe is connected, by means of a sealingly connectable conduit, to a chamber formed by a number of walls, which chamber is surrounded by a housing and communicates, by at least one wall passage, with a space defined between the housing and the chamber walls in which, by the extraction means and via suction openings communicating with the environment, an extraction flow to the remote location is generated, to which extraction flow the flow of gases coming from the exhaust pipe and fed to the chamber via the conduit, as well as the partial flow separated via the probe, are added. Through these features, the exhaust gases are passed from the exhaust pipe to a screened and properly ventilated environment and the exhaust gases are thus prevented from possibly ending up in the working environment. The specific arrangement of the chamber in the housing provides that the extraction flow does not affect the pressure of the exhaust gases, i.e. the exhaust pipe and the extraction apparatus are as it were uncoupled in terms of flow engineering, so that a soot measuring can be performed in the desired, correct manner, while on the other hand, the extraction flow along the wall passages actually provides that after measuring, the exhaust gases together with the partial flow separated therefrom are discharged to the outside environment in the desired manner.

Through the use of the conduit which can be sealingly coupled to the exhaust pipe and thus constitutes an extension of that exhaust pipe, in accordance with a further embodiment of the invention, it can be provided that the separation of the partial flow from the flow of gases by the probe takes place in the conduit. The probe can then be arranged so as to be extremely stable, for instance by fixedly connecting it to the conduit. A further advantage of this arrangement is that the probe cannot be disposed in any other manner than by coupling the extraction means to the exhaust pipe, so that, accordingly, this cannot be forgotten.

In order to enable the measuring operation to be optimally adjusted to the relevant engine, i.e. the quantity and the pressure of the gases coming from the exhaust pipe of that engine, in accordance with a further embodiment of the invention, it is provided that in the chamber, the conduit has an end having a free passage whose size is controlled depending on the quantity and/or pressure of the gases coming from the exhaust pipe and flowing to that passage.

To have in each case optimum pressure conditions in the chamber, also if the extraction apparatus is operative but the engine to be measured is not yet running, in accordance with a further embodiment of the invention, it is preferred that the chamber be provided with means for the controlled feed of ambient air.

In order to also ensure in each case optimum measuring conditions for the partial flow, it may further be provided that the partial flow separated by the probe is fed to a soot meter and, upon leaving the soot meter, is added, together with ambient air, to the extraction flow in the housing space.

Exhaust gases usually have a relatively fairly high temperature, which requires cooling of the soot meter. This can be incorporated into the system in an advantageous manner if the soot meter is cooled with ambient air that after passing the soot meter is mixed with the partial flow coming from the soot meter and subsequently discharged, together with that partial flow, through the housing via the extraction apparatus.

After the completion of the soot measurement, the vehicle engine is switched off and the fan can be allowed to continue rotating for some time for removing as many exhaust gases as possible from the housing and the chamber. To prevent the exhaust gases from ending up in the working environment as yet, by switching off the fan prematurely or when it fails unexpectedly, in accordance with a further embodiment of the invention, it can be provided that when the extraction means are switched off, the suction openings are closed off. Through such closure, it is also possible to omit after-rotation of the fan, if so desired, without this resulting in a pollution of the working environment.

Although hereinabove, the emphasis is laid in particular on a soot measurement, the method is also suitable for measuring other components in the exhaust gases. To that end, it may be provided that in the conduit, at least one further partial flow is separated from the flow of *gases,* which further partial flow can then be fed to further measuring equipment and, after being fed back to the housing, discharged by the extraction apparatus.

The invention also relates to an apparatus for performing a method as described hereinabove, i.e. an apparatus for separating a partial flow from a flow of gases coming from an exhaust pipe of an engine such as a diesel engine for a vehicle, which apparatus comprises a housing which, on one side thereof, can be brought into open communication with the environment and, on another side thereof, is connected to an extraction tube in which, by extraction means, for instance a fan, an extraction flow can be generated, and a probe having an open front end for extracting a partial flow from a flow of gases coming from the exhaust pipe. To provide, with such apparatus, a guaranteed, optimum extraction of the exhaust gases without affecting the pressure at which the gases leave the exhaust pipe, in accordance with the invention, it is proposed that within the housing, a chamber formed by walls be arranged, into which chamber one end of a conduit opens, the other end of which is located outside the housing and comprises connecting means to enable the conduit to be sealingly coupled to the exhaust pipe, the chamber walls being provided with at least one passage which brings the chamber into open communication with a space in the housing outside the chamber, through which space the extraction flow is generated and to which extraction flow the partial flow separated by the probe is also added. With such apparatus, the entire system can be made ready for operation by merely coupling the conduit to the exhaust pipe, while it is guaranteed that in each case, an optimum extraction of the exhaust gases takes place as well; certainly if it is provided that the open front end of the probe is located in the conduit.

To enable the exhaust pipe to be quickly and reliably connected to the apparatus, in accordance with a further embodiment of the invention, it may be provided that the connecting means for coupling the conduit to an exhaust pipe comprise a sleeve made from flexible, heat-resistant material and fastening means for securing the sleeve so as to clamp around the exhaust pipe with contraction. Such sleeve can readily be slid over the exhaust pipe and can quickly and reliably be sealingly adjusted to the diameter of the exhaust pipe by folding or wrinkling the sleeve together.

The fastening means can be constructed in many ways, for instance in the form of a cord laced in a seam of the sleeve. However, according to the invention, it is preferred that the fastening means consist of Velcro attached to the sleeve. In this manner, a reliable face-wise abutment of the sleeve on the exhaust pipe can be obtained.

To create conditions which, in terms of flow engineering, are as favorable as possible, in accordance with a further embodiment of the invention, it is preferred that the walls forming the chamber be arranged in the housing in such a manner that in the housing, from the open side which can be brought into open communication with the environment, longitudinal channels in the direction of the extraction tube are formed, and the part of the conduit which connects to the end in the chamber is also oriented in the direction of the extraction tube, while the open communication between chamber and housing space in the direction of the extraction tube is located beyond the end of the conduit in the chamber. The exhaust gases flowing from the conduit are then, in an advantageous manner, already oriented in the discharge direction of the extraction.

To prevent exhaust gases from possibly ending up in the working environment when the measuring proceedings have ended, the fan may be allowed to continue rotating for some time after the vehicle engine has been switched off. Also, or instead, according to the invention, it may be provided that the side of the housing which can be brought into open communication with the environment is closable by means of valves. If, in addition, the valves can be displaced by a motor between an open and a closed position, with the valves being movable into the open position when the extraction means are activated and into the closed position when they are switched off, it is also guaranteed that when the fan is switched off or fails, no exhaust gases can end up in the working environment.

To ensure in each case that optimum pressure conditions prevail in the chamber, and more in particular that no slight partial vacuum can be created therein by the extraction means, it is preferred that at least one chamber wall be provided with controllable connection openings to the environment outside the housing, whereby a longitudinal flow can be created in the chamber in the direction of the part of the conduit which connects to the end thereof in the chamber.

To prevent exhaust gases from the chamber from possibly ending up in the working environment when the fan comes to a stop, the connection openings should then be closed. This can be realized in a simple manner if the controllable connecting openings are provided in a chamber wall located within the part of the housing which is closable by the valves.

To enable adjusting the passage of the conduit to the pressure and quantity of the gases coming from the exhaust pipe, and precluding any suction action on the exhaust pipe when the engine is not running, without, however, affecting a correct measuring, in accordance with a further embodiment of the invention, it is preferred that the end of the conduit in the chamber comprise a non-return valve which is constructed and balanced so that a flow of gases passed through the conduit can open the valve substantially without loss of pressure.

A soot meter should be cleaned with some regularity.. To be able to do so in a convenient and quick manner, it is preferred that the housing be provided with means for detachably mounting a soot meter and with means for connecting the probe to the soot meter, so that the soot meter can readily be uncoupled from the housing for maintenance and the like.

Further, it is also preferred to leave the partial flow, separated by the probe and passed to the soot meter, unaffected as much as possible, as far as pressure is concerned. To this end, in accordance with a further embodiment of the invention, it is preferred that the soot meter comprise an exhaust for the gases measured, which exhaust is arranged at a housing opening communicating with the space in which the extraction flow can be generated. If it is found desirable to cool the soot meter, that same housing opening can be used therefor, by passing ambient air through the soot meter and adding that air, after leaving the soot meter, to the extraction flow via the housing opening intended.

Although only a slight quantity of exhaust gases egresses from the soot meter, that slight quantity, too, should preferably not end up in the working environment. This can be realized in a simple manner if the soot meter connects to the housing opening so as to be sealed relative to the environment, which housing opening, like the probe, can be closed by valve means when the extraction means are switched off, while by closing the probe, the last opening through which exhaust gases could escape is closed as well.

Although hereinabove, soot measuring is mainly discussed, the apparatus can likewise be used for measuring all types of other components of the exhaust gases. If, in accordance with a further embodiment of the invention, means are provided in the conduit for separating at least one further partial flow, the apparatus is even suitable for performing different measurements at the same time.

With reference to exemplary embodiments which are schematically shown in the accompanying drawings, the separating method and apparatus according to the invention will presently be further explained, by way of example only. In these drawings:
Fig. 1 is an elevational view of a separator connected to an exhaust pipe of a motor vehicle;
Fig. 2 shows, to an enlarged scale, a part of the separator shown in Fig. 1;
Fig. 3 is a side elevational view of Fig. 2;
Fig. 4 is a further possible embodiment of the separator in elevational view;
Fig. 5 is a slide elevational view of Fig. 4; and
Fig. 6 shows, in perspective and to an enlarged scale, a connecting sleeve in its starting position.

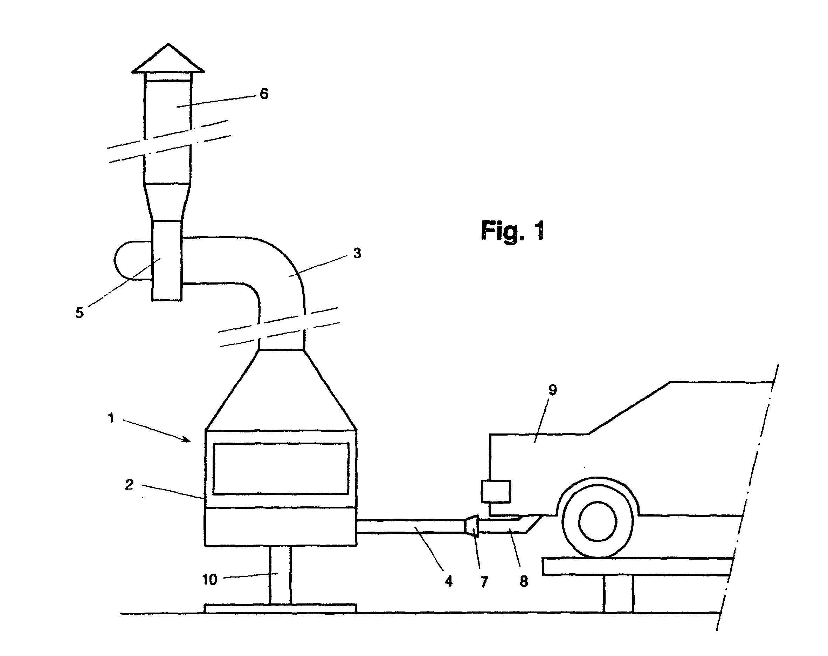

Fig. 1 shows a separator 1 comprising a housing 2 to which an extraction tube 3 connects on one side thereof, and a conduit 4 on the other. The extraction tube is connected to a fan 5, which in turn is in connection with a stack 6. Via coupling means 7, the conduit 4 is sealingly connected to an exhaust pipe 8 of a vehicle 9. The housing 2 is supported by a leg 10.

Although the arrangement seems to be rather stationary, it will be understood that the necessary flexibility can be obtained by a flexible construction of at least a part of the extraction tube 3 and the conduit 4. Further, the leg 10 may be of a mobile and height-adjustable construction.

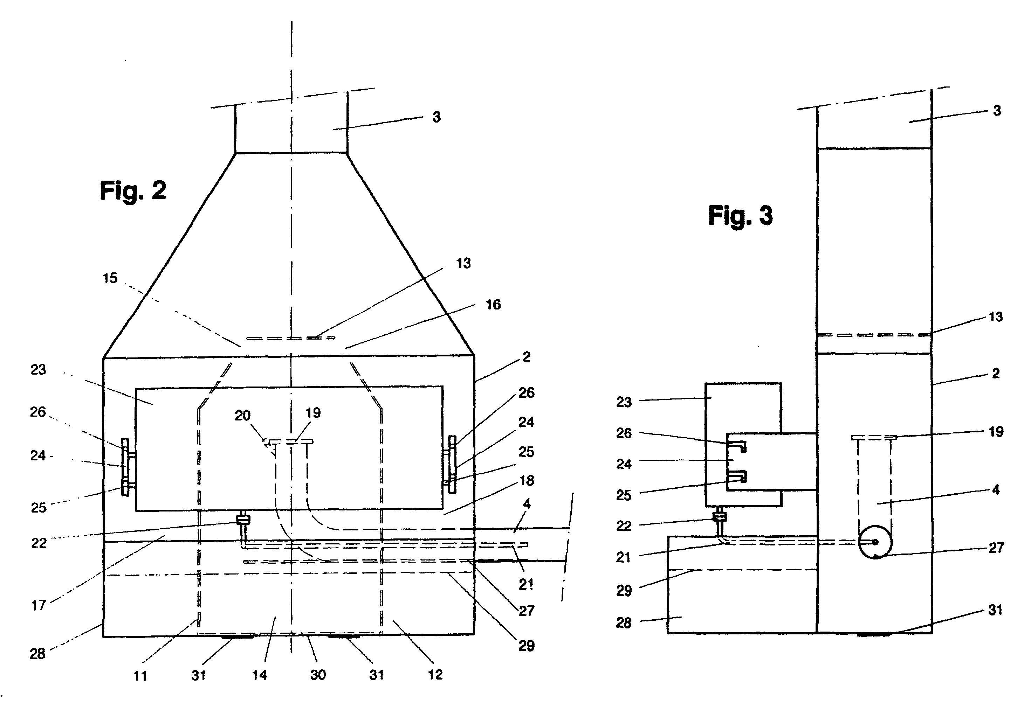

As Figs. 2 and 3 demonstrate, the housing 2 is built up from a box-shaped bottom piece and a top piece having the shape of a chimney hood and constituting a transition between the box-shaped bottom piece and the extraction tube 3. In the housing 2, a chamber 14 is formed by walls 11, 12 and 13 extending throughout the depth of the housing 2. The edges of the wall 13 which are not connected to the housing are spaced from the adjacent edges of the deflected top parts of the walls 11 and 12, to form openings 15 and 16 via which openings the chamber 14 is in open communication with the other space within the housing 2, and more in particular with two longitudinal channels 17 and 18, which are created through the arrangement of the walls 11 and 12.

The conduit 4 extends horizontally through the wall of the housing 2 and the chamber wall 12 into the chamber 14, the end of the conduit 4 in the chamber 14 being bent over at right angles to adopt a vertical position. The mouth of the conduit 4 comprises a valve 19 which is pivotable about an axis 20. The valve construction 19, 20 is of such design that normally, the valve 19 is in the closed position shown, yet opens directly at a slight excess pressure in the conduit 4, the degree of opening depending on the excess pressure and the quantity of the gases fed through the conduit 4.

Provided in the conduit 4 is a probe 21, having the shape of a tube disposed in longitudinal direction of the conduit 4 and having an open end. The probe 21 does not follow the bend of the conduit 4, but extends straight on to a position outside the conduit 4, to be subsequently bent over at right angles parallel to the wall 11. In that position, the probe 21 breaks through the wall of the housing 2 to subsequently end in a part which is bent over upwards vertically and connected, by means of a coupling 22, to a soot meter 23. Supports 24 mounted on the housing 2 support the soot meter in a readily detachable manner, for instance by means of studs 25 hooked in L-shaped slots. In the conduit 4, a further probe 27 is indicated, which can be used for feeding gases extracted from the conduit 4 to other equipment, not shown.

Below the soot meter 23, the housing 2 comprises a box-shaped built-on construction 28 which is in open communication with the longitudinal channels 17 and 18 and which comprises a perforated top wall 29. Further, the housing 2 is of open construction at its bottom side at the location of the longitudinal channels 17 and 18 and the bottom side of the chamber 14 is closed by a wall 30 comprising gratings 31 having controllable passage openings.

For performing a soot measurement, the conduit 4 is connected, by the coupling means 7, to the exhaust pipe 8 of the vehicle 9. Next, the fan 5 is started, so that ambient air is drawn in through the longitudinal channels 17 and 18 and the extraction tube 3 connected thereto, while the pressure in the chamber 14 remains substantially unaffected. In this regard, depending on the extraction conditions, it may be preferred that the gratings 31 in the bottom of the chamber 14 be slightly opened, so that any influence of the longitudinal flows in the channels 17 and 18 directed along the openings 15 and 16 on the pressure in the chamber 14 can be compensated through the inflow of ambient air by means of the gratings 31, as a result of which an atmospheric pressure prevails in that chamber 14.

Then, the engine of the vehicle 9 is started, the exhaust gases of which end up, via the exhaust pipe 8, in the conduit 4. When they reach the end of the conduit 4, the exhaust gases open the valve 19 and flow into the chamber 14, which is under atmospheric pressure; hence, this situation is comparable with the normal operating conditions of the vehicle 9, where the exhaust gases end up in the free space. The exhaust gases in the chamber 14 flow in the direction of the openings 15 and 16, where they are entrained by the longitudinal flows in the longitudinal channels 17 and 18 to the extraction tube 3 and, via this tube, passed to a remote location outside the workshop where the separator 1 is installed. In this manner, the exhaust gases are discharged as if the vehicle were not coupled to the separator 1, i.e. the flow of the exhaust gases from the exhaust pipe 8 is not affected by the presence of the separator 1; a situation required for enabling the correct measuring of the soot content of the exhaust gases.

That measuring of the soot content is effected by draining, by means of the probe 21, a partial flow from the conduit 4 and feeding this partial flow to the soot meter 23. After the measuring has been carried out, the partial flow issues from the soot meter and is then drawn, together with ambient air, via the perforated top wall 29 of the box-shaped built-on construction 28, into the housing 2 and discharged via the longitudinal channels 17 and 18 and the extraction tube 3. Further, the air issuing from the soot meter 23 is also mixed with ambient air fed through the soot meter 23 from the top side thereof for cooling the soot meter 23, which is flowed through by the relatively hot gases.

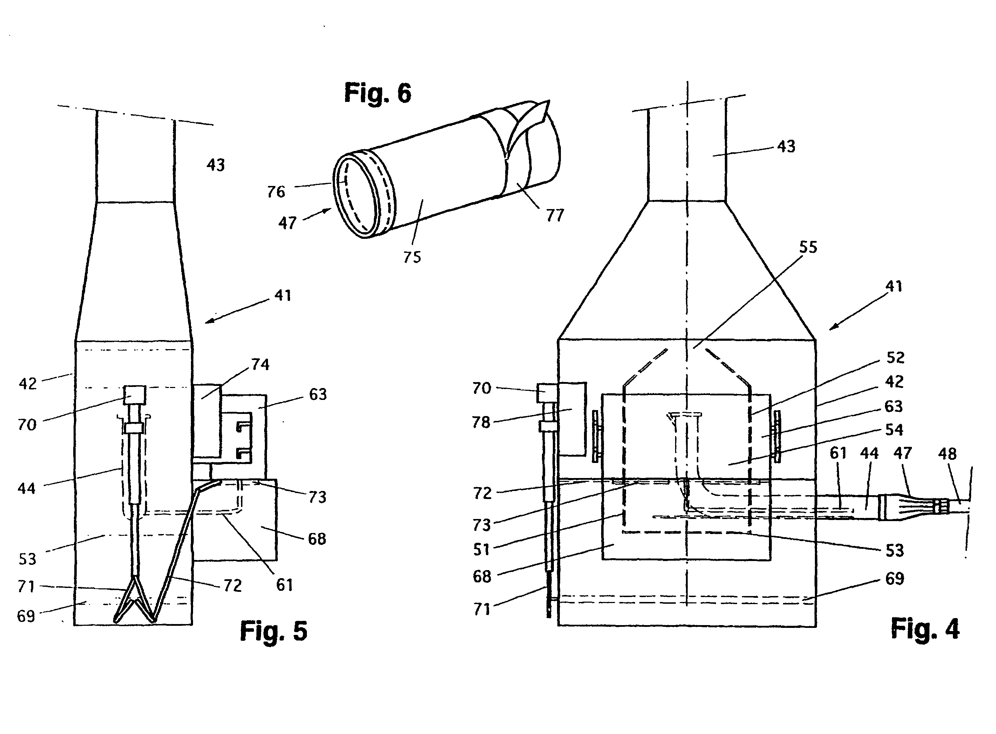

The modified embodiment shown in Figs. 4 and 5 shows a separator 41 having a housing 42 to which an extraction tube 43 connects on one side thereof and a conduit 44 on the other. The extraction tube is connected to a fan, not shown, connecting to a stack. Via a connecting sleeve 47, the conduit 44 connects to an exhaust pipe 48 of a vehicle, not further shown.

The housing 42 is built up from a box-shaped bottom piece and a top piece which has the shape of a chimney hood and which connects to the extraction tube 43. By means of walls 51, 52 and 53, extending throughout the depth of the housing 42, a chamber 54 is formed in the housing 42. The walls 51 and 52 comprise top parts deflected towards each other, whose free top edges are spaced apart to form an opening 55, via which opening the chamber 54 communicates with the other space within the housing 42.

The conduit 44 extends horizontally through the wall of the housing 42 and the chamber wall 52 into the chamber 54, the end of the conduit 44 in the chamber 54 being bent over at right angles into a vertical position. The mouth of the conduit 44 is closable by a valve in the manner as shown in Figs. 2 and 3.

Provided in the conduit 44 is a probe 61 having the shape of a tube having an open end and disposed in longitudinal direction of the conduit 44. The probe 61 ends in a soot meter 63 which is detachably connected to the housing 42.

The soot meter 63 is arranged on a box-shaped built-on construction 68 which is connected to the housing 42 and which is in open communication with the interior of the housing 42 but not with the chamber 54. Further, openings, not shown, are provided in the wall 53 of the chamber 54, which openings are closable by gratings having controllable passage openings.

The housing 42, open at its bottom side, is closable at its bottom side by means of pivoting valves 69. The pivoting valves 69 are operated by a spindle motor 70 operating a rod mechanism 71. The rod mechanism 71 also operates a further lever mechanism 72 having valves 73 which can release or close an open passage between the soot meter 63 and the box-shaped built-on construction 68. If the apparatus is not in operation, the valves 69 and 73 are in the closed position and the housing 42 is closed relative to the direct working environment, which closure can be refined even further by providing another valve in the probe 61, preferably adjacent the location where the probe 61 enters the soot meter 63.

Connecting the conduit 44 to the exhaust pipe 48 is effected by means of a connecting sleeve 47, shown to an enlarged scale in Fig. 6. The connecting sleeve 47 consists of a tubular part 75 manufactured from a flexible, heat-resistant material, one tube end being bent over and secured with a stitched seam 76. If so desired, the thus formed seam can be used for lacing in a securing cord. Adjacent its other end, the tubular part 75 is provided with Velcro 77. The diameter of the tubular part 75 is adjusted to the diameter of the conduit 44. The end of the connecting sleeve 47 having the stitching seam 76 is sealingly slid onto the conduit 44. The other end is fitted over the exhaust pipe 48 and subsequently folded or wrinkled together until the diameter of the flexible tubular part 75 is adapted to that of the exhaust pipe 48. In this position, the tubular part 75 is fixed by means of the Velcro 77, whereby in a quick and simple manner, a connection which seals reliably is obtained between the conduit 44 and the exhaust pipe 48.

When the fan is started, a control mechanism in a switch box 78 provides that the valves 69 and 73 are opened, after which the engine of the vehicle is started and the soot measuring is performed as described with reference to Figs. 2 and 3. During measuring, the extraction provides that no exhaust gases can end up in the direct working environment. When the apparatus is switched off, after the engine of the vehicle has been turned off, exhaust gases will still be present in the apparatus 41. To prevent those exhaust gases from polluting the working environment, the switching off of the fan also involves the activation of the spindle motor 70, causing the valves 69 and 73 to be moved into their closing positions and the interior of the housing 42 to be closed off relative to the working environment. When the exhaust pipe 48 is being uncoupled, the connecting sleeve 47 can be compressed completely and held in that closed-off position by means of the Velcro 77.

It is readily understood that within the framework of the invention as laid down in the appended claims, still many modifications and variants are possible. For instance, bending over the conduit 4 or 44 at right angles may be omitted, and the chamber 14 or 54 may then be positioned horizontally rather than vertically. The extraction tube 3 or 43 may then be positioned vertically upwards again, but it is also possible to arrange this extraction tube more or less horizontally in the direction of a wall of the workshop. Further, it has already been indicated that the separator can also be used for performing measurements other than a soot measurement. To that end, the partial flow separated by the probe can be used or a further partial flow which is separated by means of the further probe. In addition, a cylindrical bottom piece of the housing may also be considered, in which a cylindrical chamber is centrally arranged, so that this chamber is being flowed around by the extraction flow all over its circumference. In this arrangement, the open communication between the chamber and the other part of the housing may be located in the circumferential wall of the chamber as well as centrally in a conical top piece of the chamber. The closing of the housing when the apparatus is being switched off may be performed and controlled in any other suitable manner. For instance, non-return valves may further be arranged in the valves, enabling the fan to continue rotating for some time after the closing of the valves, so that the housing, which is then closed off for the rest, is entirely freed from exhaust gases.

## Claims

1. A method for separating, by means of a probe (21; 61), a partial flow from a flow of gases coming from an exhaust pipe (8; 48) of an engine such as a diesel engine for a vehicle (9), extraction means being present for receiving the flow of gases and discharging it to a location remote from the exhaust pipe, **characterized in that** the exhaust pipe is connected, by means of a sealingly connectable conduit (4; 44), to a chamber (14; 54) formed by a number of walls (11-13; 51-53), which chamber is surrounded by a housing (2; 42) and communicates, by at least one wall passage (15, 16; 55), with a space defined between the housing and the chamber walls in which, by the extraction means and via suction openings communicating with the environment, an extraction flow to the remote location is generated, to which extraction flow the flow of gases coming from the exhaust pipe and fed to the chamber via the conduit, as well as the partial flow separated via the probe, are added.

2. A method according to claim 1, **characterized in that** the separation of the partial flow from the flow of gases by the probe (21; 61) takes place in the conduit (4; 44).

3. A method according to claim 1 or 2, **characterized in that** in the chamber (14; 54), the conduit (4; 44) has an end having a free passage whose size is controlled depending on the quantity and/or pressure of the gases coming from the exhaust pipe (8; 48) and flowing to said passage.

4. A method according to any one of the preceding claims, **characterized in that** the chamber (14; 54) is provided with means (31) for the controlled feed of ambient air.

5. A method according to any one of the preceding claims, **characterized in that** the partial flow separated by the probe (21; 61) is fed to a soot meter (23; 63) and, upon leaving the soot meter, is added, together with ambient air, to the extraction flow in the housing space.

6. A method according to claim 5, **characterized in that** the soot meter (23; 63) is cooled with ambient air that after passing the soot meter is mixed with the partial flow coming from the soot meter.

7. A method according to any one of the preceding claims, **characterized in that** when the extraction means (5) are switched off, the suction openings are closed off.

8. A method according to any one of the preceding claims, **characterized in that** in the conduit (4; 44), at least one further partial flow is separated from the flow of gases.

9. An apparatus for separating a partial flow from a flow of gases coming from an exhaust pipe (8; 48) of an engine such as a diesel engine for a vehicle (9), said apparatus comprising a housing (2; 42) which, on one side thereof, can be brought into open communication with the environment and, on another side thereof, is connected to an extraction tube (3; 43) in which, by extraction means, for instance a fan (5), an extraction flow can be generated, and a probe (21; 61) having an open front end for extracting a partial flow from a flow of gases coming from the exhaust pipe, **characterized in that** within the housing, a chamber (14; 54) formed by walls (11-13; 51-53) is arranged, into which chamber one end of a conduit (4; 44) opens, the other end of which is located outside the housing and comprises connecting means (7; 47) to enable the conduit to be sealingly coupled to the exhaust pipe, the chamber walls being provided with at least one passage (15, 16; 55) which brings the chamber into open communication with a space in the housing outside the chamber, through which space the extraction flow is generated and to which extraction flow the partial flow separated by the probe is also added.

10. An apparatus according to claim 9, **characterized in that** the open front end of the probe (21; 61) is located in the conduit (4; 44).

11. An apparatus according to claim 9 or 10, **characterized in that** the connecting means (47) for coupling the conduit (44) to an exhaust pipe (48) comprise a sleeve (75) made from flexible, heat-resistant material and fastening means (77) for securing the sleeve so as to clamp around the exhaust pipe with contraction.

12. An apparatus according to claim 11, **characterized in that** the fastening means (77) consist of Velcro attached to the sleeve (75).

13. An apparatus according to any one of claims 9-12, **characterized in that** the walls (11-13; 51-53) forming the chamber (14; 54) are arranged in the housing (2; 42) in such a manner that in the housing, from the open side which can be brought into open communication with the environment, longitudinal channels (17, 18) in the direction of the extraction tube (3; 43) are formed, and the part of the conduit (4; 44) which connects to the end in the chamber is also oriented in the direction of the extraction tube, while the open communication (15, 16; 55) between chamber and housing space in the direction of the extraction tube is located beyond the end of the conduit in the chamber.

14. An apparatus according to any one of claims 9-13, **characterized in that** the side of the housing (2; 42) which can be brought into open communication with the environment is closable by means of valves (69).

15. An apparatus according to claim 14, **characterized in that** the valves (69) can be displaced by a motor (70) between an open and a closed position, the valves being movable into the open position when the extraction means (5) are activated and into the closed position when said extraction means are switched off.

16. An apparatus according to any one of claims 9-15, **characterized in that** at least one chamber wall (30) is provided with controllable connection openings (31) to the environment outside the housing (2), whereby a longitudinal flow can be created in the chamber (14) in the direction of the part of the conduit (4) which connects to the end thereof in the chamber.

17. An apparatus according to claim 16, **characterized in that** the controllable connecting openings (31) are provided in a chamber wall (30) located within the part of the housing (2; 42) which is closable by the valves (69).

18. An apparatus according to any one of claims 9-17, **characterized in that** the end of the conduit (4; 44) in the chamber (14; 54) comprises a non-return valve (19) which is constructed and balanced so that a flow of gases passed through the conduit can open the valve substantially without loss of pressure.

19. An apparatus according to any one of claims 9-18, **characterized in that** the housing (2; 42) is provided with means (25, 26) for detachably mounting a soot meter (23; 63) and with means for connecting the probe (21; 61) to the soot meter.

20. An apparatus according to claim 19, **characterized in that** the soot meter (23; 63) comprises an exhaust for the gases measured, said exhaust being arranged at a housing opening (29) communicating with the space in which the extraction flow can be generated.

21. An apparatus according to claim 20, **characterized in that** the soot meter (23) can be cooled by passing ambient air through it, which ambient air, after leaving the soot meter, can be passed to the extraction flow via the housing opening (29).

22. An apparatus according to claim 20 or 21, **characterized in that** the soot meter (63) connects to the housing opening so as to be sealed relative to the environment, which housing opening, like the probe (61), can be closed by valve means (73) when the extraction means (5) are switched off.

23. An apparatus according to any one of claims 9-22, **characterized in that** means (27) are provided in the conduit (4; 44) for separating at least one further partial flow.

## Patentansprüche

1. Verfahren zum mittels einer Sonde (21;61) vorgenommenen Abscheiden eines Teilstroms von einem Gasstrom, der aus dem Auspuffrohr (8;48) eines Motors wie z.B. eines Dieselmotors eines Fahrzeugs (9) austritt, wobei Extraktionsvorrichtungen vorgesehen sind, um den Gasstrom aufzunehmen und an einer von dem Auspuffrohr entfernten Stelle auszugeben,
**dadurch gekennzeichnet, dass** das Auspuffrohr mittels einer dichtend anschließbaren Leitung (4;44) mit einer durch mehrere Wände (11-13;51-53) gebildeten Kammer (14;54) verbunden ist, die von einem Gehäuse (2;42) umgeben ist und über mindestens einen Wanddurchlass (15,16; 55) mit einem zwischen dem Gehäuse und den Kammerwänden definierten Raum verbunden ist, in dem mittels der Extraktionsvorrichtungen und über mit der Umgebung kommunizierende Saugöffnungen ein Extraktionsstrom zu der entfernt gelegenen Stelle erzeugt wird, dem der aus dem Auspuffrohr austretende und der Kammer über die Leitung zugeführte Gasstrom sowie der über die Sonde getrennte Teilstrom hinzugefügt werden.

2. Verfahren nach Anspruch 1, **dadurch gekennzeichnet, dass** die mittels der Sonde (21:61) vorgenommene Abscheidung des Teilstroms von dem Gasstrom in der Leitung (4;44) erfolgt.

3. Verfahren nach Anspruch 1 oder 2, **dadurch gekennzeichnet, dass** in der Kammer (14;54) die Leitung (4;44) ein Ende mit einem freien Durchlass aufweist, dessen Größe in Abhängigkeit von der Menge und/oder dem Druck der aus dem Auspuffrohr (8;48) austretenden und zu dem Durchlass strömenden Gase gesteuert wird.

4. Verfahren nach einem der vorhergehenden Ansprüche, **dadurch gekennzeichnet, dass** die Kammer (14;54) mit einer Vorrichtung (31) zur gesteuerten Zufuhr von Umgebungsluft versehen ist.

5. Verfahren nach einem der vorhergehenden Ansprüche, **dadurch gekennzeichnet, dass** der mittels der Sonde (21;61) abgeschiedene Teilstrom einer Abgasmessvorrichtung (23;63) zugeführt wird und nach dem Verlassen der Abgasmessvorrichtung zusammen mit Umgebungsluft dem Extraktionsstrom in dem Gehäuseraum hinzugefügt wird.

6. Verfahren nach Anspruch 5, **dadurch gekennzeichnet, dass** die Abgasmessvorrichtung (23;63) mit Umgebungsluft gekühlt wird, die nach dem Passieren der Abgasmessvorrichtung mit dem von der Abgasmessvorrichtung kommenden Teilstrom gemischt wird.

7. Verfahren nach einem der vorhergehenden Ansprüche, **dadurch gekennzeichnet, dass**, wenn die Extraktionsvorrichtungen (5) ausgeschaltet sind, die Saugöffnungen geschlossen werden.

8. Verfahren nach einem der vorhergehenden Ansprüche, **dadurch gekennzeichnet, dass** in der Leitung (4;44) mindestens ein weiterer Teilstrom von dem Gasstrom abgeschieden wird.

9. Vorrichtung zum Abscheiden eines Teilstroms von einem Gasstrom, der aus dem Auspuffrohr (8;48) eines Motors wie z.B. eines Dieselmotors eines Fahrzeugs (9) austritt, mit einem Gehäuse (2;42), das an einer Seite in offene Verbindung mit der Umgebung gebracht werden kann und an einer anderen Seite mit einem Extraktionsrohr (3;43) verbunden ist, in dem durch Extraktionsvorrichtungen wie z.B. ein Gebläse (5) ein Extraktionsstrom erzeugt werden kann, und einer Sonde (21:61) mit einem offenen Vorderende zum Extrahieren eines Teilstroms aus einem Gasstrom, der aus dem Auspuffrohr austritt,
**dadurch gekennzeichnet, dass** innerhalb des Gehäuses eine durch Wände (11-13;51-53) gebildete Kammer (14;54) angeordnet ist, in die ein Ende einer Leitung (4;44) ausmündet, deren anderes Ende außerhalb des Gehäuses angeordnet ist und eine Vorrichtung (7;47) aufweist, mittels derer die Leitung dichtend mit dem Auspuffrohr verbunden werden kann, wobei die Kammerwände mit mindestens einem Durchlass (15,16;55) versehen sind, der die Kammer in offene Verbindung mit einem in dem Gehäuse außerhalb der Kammer angeordneten Raum bringt, durch den der Extraktionsstrom erzeugt wird, wobei dem Extraktionsstrom ferner der mittels der Sonde abgeschiedene Teilstrom hinzugefügt wird.

10. Vorrichtung nach Anspruch 9, **dadurch gekennzeichnet, dass** das offene Vorderende der Sonde (21;61) in der Leitung (4;44) angeordnet ist.

11. Vorrichtung nach Anspruch 9 oder 10, **dadurch gekennzeichnet, dass** die zum Verbinden de Leitung (44) mit einem Auspuffrohr 48) vorgesehene Verbindungsvorrichtung (47) eine aus flexiblem, wärmeresistentem Material gebildete Manschette (75) und eine Befestigungsvorrichtung (77) aufweist, mit der die Manschette gesichert wird, indem sie durch Kontraktion um das Auspuffrohr geklemmt wird.

12. Vorrichtung nach Anspruch 11, **dadurch gekennzeichnet, dass** die Befestigungsvorrichtung (77) einen an der Manschette (75) befestigten Klettverschluss aufweist.

13. Vorrichtung nach einem der Ansprüche 9-12, **dadurch gekennzeichnet, dass** die die Kammer (14;54) bildenden Wände (11-13;51-53) derart in dem Gehäuse (2;42) angeordnet sind, dass in dem Gehäuse von der in offene Verbindung mit der Umgebung bringbaren offenen Seite aus längsverlaufende Kanäle (17,18) in der Richtung des Extraktionsrohrs (3;43) ausgebildet sind, und der mit dem in der Kammer gelegenen Ende verbundene Teil der Leitung (4;44) ebenfalls in der Richtung des Extraktionsrohrs ausgerichtet ist, während die offene Verbindung (15,16;55) zwischen der Kammer und dem Gehäuseraum in der Richtung des Extraktionsrohrs hinter dem in der Kammer befindlichen Ende der Leitung angeordnet ist.

14. Vorrichtung nach einem der Ansprüche 9-13, **dadurch gekennzeichnet, dass** die in offene Verbindung mit der Umgebung bringbare Seite des Gehäuses (2;42) durch Ventile (69) schließbar ist.

15. Vorrichtung nach Anspruch 14, **dadurch gekennzeichnet, dass** die Ventile (69) mittels eines Motors (70) zwischen einer offenen und einer geschlossene Position bewegt werden können, wobei die Ventile in die offene Position bewegbar sind, wenn die Extraktionsvorrichtungen (5) aktiviert sind, und in die geschlossene Position bewegbar sind, wenn die Extraktionsvorrichtungen ausgeschaltet sind.

16. Vorrichtung nach einem der Ansprüche 9-15, **dadurch gekennzeichnet, dass** mindestens eine Kammerwand (30) mit steuerbaren Verbindungsöffnungen (31) zu der Umgebung außerhalb des Gehäuses (2) versehen ist, wobei in der Kammer (14) ein Längsstrom in der Richtung desjenigen Teils der Leitung (4) erzeugt werden kann, der in Verbindung mit dem in der Kammer gelegenen Ende der Leitung steht.

17. Vorrichtung nach Anspruch 16, **dadurch gekennzeichnet, dass** die steuerbaren Verbindungsöffnungen (31) in einer Kammerwand (30) ausgebildet sind, die in demjenigen Teil des Gehäuses (2;42) angeordnet ist, der durch die Ventile (69) schließbar ist.

18. Vorrichtung nach einem der Ansprüche 9-17, **dadurch gekennzeichnet, dass** das in der Kammer (14;54) befindliche Ende der Leitung (4;44) ein Rückschlagventil (19) aufweist, das derart ausgebildet und abgeglichen ist, dass der durch die Leitung geführte Gasstrom das Ventil im wesentlichen ohne Druckverlust öffnen kann.

19. Vorrichtung nach einem der Ansprüche 9-18, **dadurch gekennzeichnet, dass** das Gehäuse (2;42) mit einer Vorrichtung (25,26) zur abnehmbaren Befestigung einer Abgasmessvorrichtung (23;63) und mit einer Vorrichtung zum Verbinden der Sonde (21;61) mit der Abgasmessvorrichtung versehen ist.

20. Vorrichtung nach Anspruch 19, **dadurch gekennzeichnet, dass** die Abgasmessvorrichtung (23;63) einen Auslass für die zu messenden Gase aufweist, wobei der Auslass an einer Gehäuseöffnung (29) angeordnet ist, die mit dem Raum verbunden ist, in dem der Extraktionsstrom erzeugt werden kann.

21. Vorrichtung nach Anspruch 20, **dadurch gekennzeichnet, dass** die Abgasmessvorrichtung (23) gekühlt werden kann, indem Umgebungsluft durch sie hindurchgeleitet wird, wobei die Umgebungsluft nach dem Verlassen der Abgasmessvorrichtung über die Gehäuseöffnung (29) dem Extraktionsstrom zugeführt werden kann.

22. Vorrichtung nach Anspruch 20 oder 21, **dadurch gekennzeichnet, dass** die Abgasmessvorrichtung (63) derart mit der Gehäuseöffnung verbunden ist, dass sie der Umgebung gegenüber abgedichtet ist, wobei die Gehäuseöffnung wie die Sonde (61) durch Ventilvorrichtungen (73) geschlossen werden kann, wenn die Extraktionsvorrichtungen (5) ausgeschaltet sind.

23. Vorrichtung nach einem der Ansprüche 9-22, **dadurch gekennzeichnet, dass** in der Leitung (4;44) Vorrichtungen (27) zum Abscheiden mindestens eines weiteren Teilstroms vorgesehen sind.

## Revendications

1. Procédé de séparation, à l'aide d'une sonde (21 ; 61), d'un courant partiel à partir d'un courant de gaz provenant d'une tubulure d'échappement (8 ; 48) d'un moteur à combustion interne, tel qu'un moteur diesel destiné à un véhicule (9), un dispositif d'extraction étant présent afin qu'il reçoive le courant de gaz et l'évacue vers un emplacement distant de la tubulure d'échappement, **caractérisé en ce que** la tubulure d'échappement est raccordée, par un conduit (4 ; 44) permettant un raccordement étanche, à une chambre (14 ; 54) formée par un certain nombre de parois (11-13 ; 51-53), la chambre étant entourée par un boîtier (2 ; 42) et communiquant, par au moins un passage de paroi (15 ; 16 ; 55), avec un espace délimité entre le boîtier et les parois de la chambre et dans lequel, par le dispositif d'extraction et les ouvertures d'aspiration communiquant avec le milieu, un courant d'extraction vers l'emplacement distant est créé, et le courant de gaz provenant de la tubulure d'échappement et transmis à la chambre par le conduit ainsi que le courant partiel séparé par la sonde sont ajoutés au courant d'extraction.

2. Procédé selon la revendication 1, **caractérisé en ce que** la séparation du courant partiel du courant de gaz par la sonde (21 ; 61) est réalisée dans le conduit (4 ; 44).

3. Procédé selon la revendication 1 ou 2, **caractérisé en ce que**, dans la chambre (14 ; 54), le conduit (4 ; 44) a une extrémité ayant un passage libre dont la dimension est réglée d'après la quantité et/ou la pression des gaz provenant de la tubulure d'échappement (8 ; 48) et circulant vers ledit passage.

4. Procédé selon l'une quelconque des revendications précédentes, **caractérisé en ce que** la chambre (14 ; 54) comporte un dispositif (31) de transmission réglée d'air ambiant.

5. Procédé selon l'une quelconque des revendications précédentes, **caractérisé en ce que** le courant partiel séparé par la sonde (21 ; 61) est transmis à un organe de mesure de suie (23 ; 63) et, après avoir quitté l'organe de mesure de suie, est ajouté, avec de l'air ambiant, au courant d'extraction dans l'espace du boîtier.

6. Procédé selon la revendication 5, **caractérisé en ce que** l'organe de mesure de suie (23 ; 63) est refroidi par de l'air ambiant qui, après passage dans l'organe de mesure de suie, se mélange au courant partiel provenant de l'organe de mesure de suie.

7. Procédé selon l'une quelconque des revendications précédentes, **caractérisé en ce que**, lorsque le dispositif d'extraction (5) est commuté à l'arrêt, les ouvertures d'aspiration sont fermées.

8. Procédé selon l'une quelconque des revendications précédentes, **caractérisé en ce que**, dans le conduit (4 ; 44), un courant partiel supplémentaire au moins est séparé du courant de gaz.

9. Appareil de séparation d'un courant partiel d'un courant de gaz provenant d'une tubulure d'échappement (8 ; 48) d'un moteur à combustion interne, tel qu'un moteur diesel destiné à un véhicule (9), l'appareil comprenant un boîtier (2 ; 42) qui, d'un premier côté, peut être mis en communication libre avec le milieu et, d'un autre côté, est raccordé a un tube d'extraction (3 ; 43) dans lequel, par un dispositif d'extraction, par exemple un ventilateur (5), un courant d'extraction peut être créé, et une sonde (21 ; 61) ayant une extrémité avant ouverte pour l'extraction d'un courant partiel d'un courant de gaz provenant de la tubulure d'échappement,
**caractérisé en ce que**, à l'intérieur du boîtier, une chambre (14 ; 54) est disposée et formée par des parois (11-13 ; 51-53), une première extrémité d'un conduit (4 ; 44) débouchant dans cette chambre, l'autre extrémité du conduit étant placée à l'extérieur du boîtier et comprenant un dispositif de raccordement (7 ; 47) destiné à permettre au conduit d'être couplé de manière étanche à la tubulure d'échappement, les parois de la chambre ayant au moins un passage (15 ; 16 ; 55) qui met la chambre en communication libre avec un espace dans le boîtier à l'extérieur de la chambre, le courant d'extraction étant créé par l'intermédiaire de cet espace et le courant partiel séparé par la sonde étant aussi ajouté au courant d'extraction.

10. Appareil selon la revendication 9, **caractérisé en ce que** l'extrémité avant ouverte de la sonde (21 ; 61) est disposée dans le conduit (4 ; 44).

11. Appareil selon la revendication 9 ou 10, **caractérisé en ce que** le dispositif de raccordement (47) destiné à coupler le conduit (44) à une tubulure d'échappement (48) comporte un manchon (75) formé d'un matériau flexible résistant à la chaleur, et un dispositif de fixation (77) destiné à fixer le manchon afin qu'il assure le serrage autour de la tubulure d'échappement avec contraction.

12. Appareil selon la revendication 11, **caractérisé en ce que** le dispositif de fixation (77) est constitué de "Velcro" fixé au manchon (75).

13. Appareil selon l'une quelconque des revendications 9 à 12, **caractérisé en ce que** les parois (11-13 ; 51-53) formant la chambre (14 ; 54) sont disposées dans le boîtier (2 ; 42) de manière que, dans le boîtier, depuis le côté ouvert qui peut être mis en communication libre avec le milieu, des canaux longitudinaux (17, 18) dans la direction du tube d'extraction (3 ; 43) soient formés, et la partie du conduit (4 ; 44) qui se raccorde à l'extrémité de la chambre soit aussi orientée dans la direction du tube d'extraction, alors que la communication libre (15 ; 16 ; 55) entre la chambre et l'espace du boîtier dans la direction du tube d'extraction se trouve au-delà de l'extrémité du conduit dans la chambre.

14. Appareil selon l'une quelconque des revendications 9 à 13, **caractérisé en ce que** le côté du boîtier (2 ; 42) qui peut être mis en communication libre avec le milieu peut être fermé par des soupapes (69).

15. Appareil selon la revendication 14, **caractérisé en ce que** les soupapes (69) peuvent être déplacées par un moteur (70) entre des positions d'ouverture et de fermeture, les soupapes étant mobiles vers la position d'ouverture lorsque le dispositif d'extraction (5) est activé et vers la position de fermeture lorsque le dispositif d'extraction est mis à l'arrêt.

16. Appareil selon l'une quelconque des revendications 9 à 15, **caractérisé en ce qu'**au moins une paroi (30) de la chambre a des ouvertures réglables de raccordement (31) au milieu en dehors du boîtier (2), si bien qu'un courant longitudinal peut être créé dans la chambre (14) dans la direction de la partie du conduit (4) qui se raccorde à son extrémité dans la chambre.

17. Appareil selon la revendication 16, **caractérisé en ce que** les ouvertures réglables de raccordement (31) sont disposées dans une paroi de chambre (30) disposée dans la partie du boîtier (2 ; 42) qui peut être fermée par les soupapes (69).

18. Appareil selon l'une quelconque des revendications 9 à 17, **caractérisé en ce que** l'extrémité du conduit (4 ; 44) placée dans la chambre (14 ; 54) comporte un clapet de retenue (19) dont la construction et l'équilibre sont tels qu'un courant de gaz transmis dans le conduit peut ouvrir le clapet pratiquement sans perte de charge.

19. Appareil selon l'une quelconque des revendications 9 à 18, **caractérisé en ce que** le boîtier (2 ; 42) comporte un dispositif (25, 26) de montage amovible d'un organe de mesure de suie (23 ; 63) et un dispositif de raccordement de la sonde (21 ; 61) à l'organe de mesure de suie.

20. Appareil selon la revendication 19, **caractérisé en ce que** l'organe de mesure de suie (23 ; 63) comporte un organe d'échappement des gaz mesurés, l'organe d'échappement étant placé à une ouverture (29) du boîtier qui communique avec l'espace dans lequel peut être créé le courant d'extraction.

21. Appareil selon la revendication 20, **caractérisé en ce que** l'organe de mesure de suie (23) peut être refroidi par circulation d'air ambiant à l'intérieur, l'air ambiant, lorsqu'il a quitté l'organe de mesure de suie, pouvant passer vers le courant d'extraction par l'intermédiaire de l'ouverture de boîtier (29).

22. Appareil selon la revendication 20 ou 21, **caractérisé en ce que** l'organe de mesure de suie (63) est raccordé à l'ouverture du boîtier afin qu'il soit enfermé de manière étanche par rapport au milieu environnant, et l'ouverture du boîtier, comme la sonde (61), peut être fermée par un dispositif à soupape (73) lorsque le dispositif d'extraction (5) est commuté à l'arrêt.

23. Appareil selon l'une quelconque des revendications 9 à 22, **caractérisé en ce qu'**un dispositif (27) est placé dans le conduit (4 ; 44) de séparation d'au moins un courant partiel supplémentaire.
